Europäisches Patentamt

European Patent Office

Office européen des brevets

(11) Veröffentlichungsnummer: **0 184 765 B1**

# EUROPÄISCHE PATENTSCHRIFT

(45) Veröffentlichungstag der Patentschrift: **02.05.91**

(51) Int. Cl.5: **G01N 33/96**, //G01N33/92

(21) Anmeldenummer: **85115384.1**

(22) Anmeldetag: **04.12.85**

(54) Kontroll- bzw. Eichserum für die Lipid-Diagnostik.

(30) Priorität: **10.12.84 DE 3445010**

(43) Veröffentlichungstag der Anmeldung:
**18.06.86 Patentblatt 86/25**

(45) Bekanntmachung des Hinweises auf die
Patenterteilung:
**02.05.91 Patentblatt 91/18**

(84) Benannte Vertragsstaaten:
**AT BE CH DE FR GB IT LI LU NL SE**

(56) Entgegenhaltungen:
**EP-A- 0 009 596       EP-A- 0 031 955
EP-A- 0 035 211       EP-A- 0 058 959
US-A- 3 876 375       US-A- 3 955 925**

**Pschyrembel, Klinisches Wörterbuch, Verlag
de Gruyter, Berlin, 1986, 255. Auflage, Seiten
104, 183, 278**

(73) Patentinhaber: **BOEHRINGER MANNHEIM
GMBH
Sandhofer Strasse 116
W-6800 Mannheim 31(DE)**

(72) Erfinder: **Portenhauser, Rudolf, Dr. rer. nat.
Kustermannstrasse 26 b
W-8132 Tutzing(DE)**
Erfinder: **Bartl, Knut, Dr. rer. nat.
Am Westend 6
W-8121 Wilzhofen(DE)**

**Beschreibung**

Gegenstand der Erfindung ist ein Kontroll- bzw. ein Eichserum, das für die Lipid-Diagnostik relevante Parameter in einer einzigen, lagerstabiler Form enthält, sowie ein Verfahren zu dessen Herstellung.

Neuere biochemische Erkenntnisse haben wesentlich zur Aufklärung von Störungen des Fett- und Lipoproteinstoffwechsels beigetragen. Eine verfeinerte Lipoproteinanalytik erlaubt Korrelationen zwischen der Konzentration einzelner Parameter und der Wahrscheinlichkeit koronarer Erkrankungen bzw. des Risikos für das Eintreten eines Infarktes. Besondere Bedeutung hat in diesem Zusammenhang die Bestimmung des Gesamtcholesterins, des HDL-Cholesterins und des LDL-Cholesterins erlangt. Jedoch ist auch die Bestimmung der anderen Parameter, wie Triglyceride, Apolipoproteine A1, A2 und B, von Bedeutung.

Zur Richtigkeits- und Präzisionskontrolle von Bestimmungsmethoden bzw. zum Eichen von Analysenautomaten werden üblicherweise Kontrollseren bzw. Eichseren eingesetzt, welche die zu bestimmenden Parameter in bekannten, genau festgelegten Konzentrationen enthalten. Man unterscheidet Spezialkontrollseren, die zur Kontrolle bzw. zur Eichung von ganz bestimmten Parametern oder einer begrenzten, meist zusammengehörigen Gruppe von Parametern dienen, und sogenannte Universalkontrollseren, die zur Kontrolle bzw. zum Eichen möglichst aller gängigen Parameter nach sämtlichen, in der Praxis üblicherweise durchgeführten Bestimmungsmethoden einsetzbar sind.

Die Anforderungen, die an solche Kontroll- bzw. Eichseren im klinisch-chemischen Routinebetrieb gestellt werden, umfassen:

- Bei Kontrollseren müssen die Konzentrationsbereiche der einzelnen Meßparameter genau bekannt sein, und bei Eichseren muß eine genau bekannte Konzentration des bestimmten Meßparameters eingehalten werden.
- Die Konzentrationen müssen im medizinisch relevanten Meßbereich (normal bzw. pathologisch) liegen.
- Die Handhabung dieser Seren muß einfach sein.
- Die Seren müssen eine möglichst lange Haltbarkeit besitzen.

Für die Lipid-Diagnostik sind Kontroll- bzw. Eichseren seit einiger Zeit bekannt. Ein Nachteil dieser bekannten Seren ist, daß sie jeweils nur für ganz bestimmte Lipid-Parameter geeignet sind. Ein Kontrollserum bzw. Eichserum, das sämtliche oder mindestens alle relevanten Parameter für die Lipid-Diagnostik enthält, ist bisher nicht bekannt.

So kann man beispielsweise Kontrollseren für die Bestimmung von HDL-Cholesterin und die Apo-Lipoproteine käuflich erwerben. Ein Qualitätskontroll- bzw. Eichserum, das neben diesen Parametern auch LDL-Cholesterin in hinreichender Konzentration enthält, ist bisher nicht beschrieben worden.

Gerade dieser Parameter ist jedoch für die Diagnostik von besonderer Bedeutung. Bereits 1954 wurde durch Auftrennen der Lipoproteine in der Ultrazentrifuge gefunden, daß bei vorliegen koronarer Herzkrankheiten häufig hohe LDL-Konzentrationen gemessen werden. Von Jenkins et al. (Brit. Med. Journal 2 [1978], S. 388 ff.) konnte der Zusammenhang zwischen der Schwere einer Koronarkrankheit und der Höhe der LDL-Cholesterin-Konzentration aufgezeigt werden. Obwohl die Wichtigkeit der LDL-Bestimmung schon lange erkannt worden ist, gab es bisher kein Kontroll- bzw. Eichserum, das die für die Lipid-Diagnostik relevanten Parameter, insbesondere auch LDL-Cholesterin, in der erforderlichen Stabilität und den erforderlichen Konzentrationen in einer einzigen Darreichungsform enthält.

In US-A-3,955,925 wird ein optisch klares Serum beschrieben, das zur Herstellung von Standards und Qualitätskontrollmaterialien für die Bestimmung menschlicher Serumkomponenten geeignet ist. Das optisch klare Serum wird dadurch erhalten, daß aus menschlichem Serum Chylomikronen, VLDL- und LDL-Lipoproteine durch selektive Fällung entfernt werden. Möglicherweise durch eine solche Fällung gesenkte Lipid-, Gesamtcholesterin- und Triglyceridspiegel werden durch Zugabe löslicher Derivate oder lyophilisierbarer Lipoproteine aus tierischen Quellen wieder in den ursprünglichen Konzentrationsbereich gebracht. Das resultierende optisch klare Serum enthält jedoch keine Chylomikronen, kein VLDL-Lipoprotein und kein LDL-Lipoprotein. Es fehlen damit wichtige Komponenten, die relevant sind für die Lipid-Diagnose im normalen und pathologischen menschlichen Konzentrationsbereich.

EP-A-O 009 596 betrifft eine wässrige Lipid-Standardlösung, die einem Serum nahekommen soll, die aber eben kein Serum ist. Die Lösung enthält Cholesterin und/oder Triolein, nichtionisches und ionisches Detergens sowie gegebenenfalls Cholesterinester langkettiger Fettsäuren. Für eine differenzierte Lipiddiagnostik notwendige Komponenten, wie HDL- und LDL-Cholesterin sowie Apolipoproteine A1, A2 und B enthält die beschriebene wässrige Lipid-Standardlösung nicht.

Aus EP-A-O 031 955 ist ein verfahren zur Isolierung von Cholesterin aus Plasma oder Serum bekannt. So isoliertes Cholesterin wird, gelöst in destilliertem oder deionisiertem Wasser, als Kontrollösung für Cholesterin eingesetzt. Außer Cholesterin sind in dieser wässrigen Lösung keine anderen für die Lipid-Diagnostik wichtigen Parameter enthalten.

Es bestand daher weiterhin der Bedarf an einem Kontroll- bzw. Eichserum, das bei der Bestimmung jedes der für die Lipid-Diagnostik wichtigen Parameters eingesetzt werden kann.

Aufgabe der Erfindung war es, diesen Bedarf zu befriedigen. Die Aufgabe wird durch das erfindungsgemäße Kontroll- und Eichserum gelöst.

Gegenstand der Erfindung ist somit ein Kontroll- bzw. Eichserum, das die für die Lipid-Diagnostik relevanten Parameter im normalen oder pathologischen Konzentrationsbereich in einer einzigen, lagerstabilen Darreichungsform enthält.

Das erfindungsgemäße Kontroll- bzw. Eichserum enthält vorzugsweise die für den Fettstoffwechsel wichtigen Parameter, Gesamtcholesterin, HDL-Cholesterin, LDL-Cholesterin Triglyceride sowie die drei Apolipoproteine A1, A2 und B menschlichen Ursprungs. Daneben können auch ß-Lipoproteine, Phospholipide und Lecithin enthalten sein. Mindestens die Apolipoproteine A1, A2 und B sind Bestandteil eines Kontroll- bzw. Eichserums gemäß der Erfindung. Besonders bevorzugt ist ein Kontroll- bzw. Eichserum, das mindestens für die Parameter HDL-Cholesterin, LDL-Cholesterin und die Apolipoproteine A1, A2 und B geeignet ist.

In einem Kontrollserum bewegen sich Konzentrationbereiche der einzelnen Parameter in den in Tabelle 1 angegebenen Grenzen, wobei zwischen einem Kontrollserum für den normalen Bereich und für den pathologischen Bereich unterschieden werden muß.

## Tabelle 1

## Konzentrationsbereiche für die einzelnen Parameter eines Kontrollserums

| Substanz | normaler Bereich [mg/dl) | pathologischer Bereich [mg/dl] |
|---|---|---|
| Gesamtcholesterin | 140 - 240 | 220 - 320 |
| HDL-Cholesterin | 50 - 70 | 20 - 50 |
| LDL-Cholesterin | 90 - 150 | 140 - 250 |
| Triglyceride | 70 - 110 | 120 - 200 |
| Apo A1 | 90 - 140 | 50 - 90 |
| Apo A2 | 25 - 50 | 20 - 40 |
| Apo B | 80 - 120 | 110 - 170 |
| ß-Lipoproteine | 350 - 650 | |
| Phospholipide | 150 - 300 | |
| Lecithin | 120 - 220 | |

Die Eichseren enthalten die einzelnen Parameter in einer ganz bestimmten Konzentration. Diese Konzentration kann selbstverständlich von Charge zu Charge in gewissen Grenzen variieren. Die Werte liegen üblicherweise in dem in Tabelle 2 angegebenen Bereich.

Tabelle 2

Konzentrationsbereiche für Eichseren

| Substanz | Konzentration [mg/dl] |
|----------|------------------------|
| Apolipoprotein A1 | 100 - 130 |
| Apolipoprotein A2 | 35 - 50 |
| Apolipoprotein B | 85 - 115 |
| Gesamtcholesterin | 180 - 280 |
| HDL-Cholesterin | 40 - 80 |
| LDL-Cholesterin | 120 - 180 |
| Triglyceride | 90 - 130 |

Sowohl das erfindungsgemäße Kontrollserum als auch das Eichserum können zusätzliche Hilfsstoffe enthalten, beispielsweise Aufhellungsmittel, Stabilisierungsmittel, Detergentien und Konservierungsstoffe. Als Aufhellungsmittel ist beispielsweise Pentaerythrit, als Stabilisierungsmittel sind vor allem Zucker, insbesondere Saccharose, geeignet. Als Konservierungstoffe können vor allem Azide, Merthiolat und Antibiotika dienen. Jedoch sind auch andere, dem Fachmann bekannte Hilfsstoffe einsetzbar.

Zur Herstellung des erfindungsgemäßen Kontroll- bzw. Eichserums geht man von frischem Humanserum aus. Man kann auch frisches Humanplasma benutzen, das zu Humanserum umgearbeitet wird. Das Humanplasma bzw. -serum soll nicht älter als 5 bis 10 Tage sein. Auch Humanplasma bzw. Humanserum, das unmittelbar nach Gewinnung tiefgefroren wurde und in dieser Form zwischenlagerbar ist, kann zur Herstellung des erfindungsgemäßen Produkts verwendet werden. Das Humanplasma wird dabei nach dem Auftauen zu Humanserum umgearbeitet. Im Rohstoff wird in üblicher Weise die gewünschte Parameter-Konzentration eingestellt. Dies kann durch Einengen der Lösung erfolgen, wenn eine höhere Konzentration der Inhaltsstoffe erreicht werden soll. Ist es erforderlich, die Parameter-Konzentration zu erniedrigen, so wird ein Verdünnungsmittel, beispielsweise eine geeignete Pufferlösung zugesetzt. Es werden die Hilfsstoffe,beispielsweise 4 - 12 Gewichtsprozent eines Stabilisierungsmittels, wie Saccharose, zugegeben. Danach erfolgt zur Erniedrigung der Keimzahl eine Filtration über einen Membranfilter (≦2 µm Maschenweite). Die so gewonnene Lösung wird in der gewünschten Menge in Fläschchen geeigneter Größe (1 bis 5 ml) abgefüllt und vorzugsweise lyophilisiert.

Das erfindungsgemäße Kontroll- bzw. Eichserum kann auch auf der Basis von Rinderserumalbumin (RSA) erhalten werden. Hierzu ist es erforderlich, durch Zusatz geeigneter Konzentrate die gewünschte parameterkonzentration einzustellen. Als solche Konzentrate eignen sich insbesondere HDL-Cholesterin- und LDL-Cholesterin-Konzentrate menschlichen Ursprungs, triglyceridreiche Eigelbextrakte usw. Das Rinderserumalbumin, bevorzugt in Form einer wäßrigen Lösung (6 - 10 Gewichtsprozent), wird mit den Konzentraten vermischt. Durch Variation der Mischungsverhältnisse können in gewünschter Weise "normale" und "pathologische" Konzentrationen der Lipidparameter erhalten werden.

Das erfindungsgemäße Kontroll- bzw. Eichserum ist im lyophilisierten Zustand bei 4° C mindestens zwei Jahre haltbar. Es eignet sich hervorragend als Qualitätskontroll- und Eichserum für HDL-Cholesterin, LDL-Cholesterin und drei Apo-Lipoproteine A1, A2 und B. Insbesondere lassen sich erfindungsgemäß die drei Lipoproteine A1, A2 und B in genügend hohen Konzentrationen anreichern, daß es ohne weiteres möglich ist, Eichkurven sowohl für den relevanten pathologischen und den physiologischen Bereich zu erstellen.

Die Erfindung wird durch die folgenden Beispiele näher erläutert.

Beispiel 1

Qualitätskontrollserum für die Lipidkontrolle (normal)

600 ml frisches Humanplasma, Proteingehalt 5.0 g/dl, wird durch Recalcifizieren und Abtrennen des Gerinnungskuchens zu Humanserum umgearbeitet. Das Serum wird auf die Hälfte seines ursprünglichen Volumens (auf ca. 300 ml) eingeengt und damit gleichzeitig die Proteinkonzentration auf 7,7 g/dl gebracht.

Nach Dialyse (3 x 5 Std.) gegen jeweils das 30fache Volumen eines Puffers pH 8.0 (Puffer enthält KCl 4.5 mmol/l, NaCl 105 mmol/l, $MgSO_4.7H_2O$ I mmol/l, $Na_2HPO_4.2H_2O$ I mmol/l, $NaHCO_3$ 15 mmol/l, Na-Acetat 17.5 mmol/l) wird das Serum zur Abreicherung von Keimen filtriert (EKS I - Filter der Firma Schleicher & Schüll). Die Ausbeute an Serum nach dieser Filtration beträgt 250 ml. Es werden 10 Gew.-% Saccharose (10 g/100 ml) darin gelöst und das Serum durch Membranfilter mit $\leq$ 0,2 $\mu$m Maschenweite filtriert. Abschließend wird das Serum in 3 ml-Portionen in Fläschchen eingefüllt und lyophilisiert.

Das so gewonnene Lyophilisat enthält nach Rekonstitution mit 3 ml $H_2O$ bidest. die folgenden Bestandteile in den angegebenen Konzentrationen:

| | |
|---|---|
| Gesamtcholesterin | 231,2 mg/dl |
| HDL-Cholesterin | 65,7 mg/dl |
| LDL-Cholesterin | 144,7 mg/dl |
| Triglyceride | 106,1 mg/dl |
| Apo A1 | 105,6 mg/dl |
| Apo A2 | 41,3 mg/dl |
| Apo B | 99,1 mg/dl |
| ß-Lipoproteine | 556,0 mg/dl |
| Phospholipide | 276,0 mg/dl |
| Lecithin | 201,0 mg/dl |

Beispiel 2

Qualitätskontrollserum für die Lipidkontrolle (pathologisch)

500 ml frisches Humanplasma, Proteingehalt 5.7 g/dl, wurden wie in Beispiel 1 beschrieben, zu Serum umgearbeitet. Das Serum wurde auf 225 ml eingeengt und wies nach Einengung und Dialyse (wie in Beisp. 1) einen Proteingehalt von 9.8 g/dl auf. Zur Abreicherung von Keimen wurde das Serum durch EKS I-Filter (Schleicher & Schüll) filtriert. Ausbeute nach dieser Filtration 200 ml Serum.

Zur Erhöhung der LDL-Cholesterin- und der Apolipoprotein-B-Konzentration wurden die 200 ml Serum mit 95 ml eines Human-LDL-Cholesterin-Konzentrates (Herstellung siehe unten) versetzt und gemischt.

Es wurden 10 Gewichtsprozent Saccharaose (10 g/100 ml) darin gelöst und das Serum durch Membranfilter mit $\leq$ 0.2 $\mu$m Maschenweite filtriert. Abschließend wird das Serum in 3-ml-Portionen in Fläschchen gefüllt und lyophilisiert.

Das so gewonnene Lyophilisat enthält nach Rekonstitution mit 3 ml $H_2O$ bidest. die folgenden Bestandteile in den angegebenen Konzentrationen:

| Gesamtcholesterin | 314.0 mg/dl |
|---|---|
| HDL-Cholesterin | 47.8 mg/dl |
| LDL-Cholesterin | 234.9 mg/dl |
| Triglyceride | 157.3 mg/dl |
| Apo A1 | 85.7 mg/dl |
| Apo A2 | 32.0 mg/dl |
| Apo B | 165.2 mg/dl |
| ß-Lipoproteine | nicht gemessen |
| Phospholipide | nicht gemessen |
| Lecithin | nicht gemessen. |

Zur Herstellung des Human-LDL-Cholesterin-Konzentrates wird Humanplasma in bekannter Weise zu Humanserum umgearbeitet und gegen den in Beispiel 1 beschriebenen Puffer dialysiert. Aus 2500 ml so gewonnenem Humanserum wird mit Polyvinylsulfatlösung (c = 3 g/l; Menge 500 ml) LDL-Cholesterin ausgefällt und das Sediment in 100 ml einer 0.05 %igen EDTA-Lösung mit 4.6 % NaCl, pH 7.0 aufgenommen und gelöst. Ausbeute 135 ml. Diese Lösung wird über Nacht gegen 10 1 3 M KCl in 0.05 % EDTA, pH 7.0 dialysiert und vom gebildeten Niederschlag abzentrifugiert. Der überstand, 110 ml, wird zur Entfernung des KCl gegen 10 1 einer Lösung von NaCl 120 mmol/1 und NaHCO₃ 25 mmol/l dialysiert.

Die Ausbeute beträgt 130 ml mit folgenden Analytkonzentrationen:

| Gesamtcholesterin | 1004.0 mg/dl |
|---|---|
| HDL-Cholesterin | 4.3 mg/dl |
| LDL-Cholesterin | 902.0 mg/dl |

Dieses Human-LDL-Cholesterin-Konzentrat wird mit 0.1 Gew.-% Natrium-Azid stabilisiert und bis zur verwendung bei 4 °C aufbewahrt.

Beispiel 3

Eichserum für die Apolipoproteine A1, A2 und B

500 ml frisches Humanplasma mit einem Proteingehalt von 5.6 g/dl wird wie in Beispiel 1 beschrieben zu Serum umgearbeitet und so weit eingeengt (auf ca. 270 ml), daß ein Proteingehalt von 9.0 g/dl resultiert. Es folgt die Dialyse und die Filtration durch EKS-I-Filter, wie in Beispiel 1 angegeben. Serumausbeute beträgt hier 220 ml.

Es werden 10 Gewichtsprozent Saccharose (10 g/100 ml) darin gelöst und das Serum durch Membranfilter mit 0.2μm Maschenweite filtriert. Abschließend wird das Serum in 1-ml-Portionen in Fläschchen gefüllt und lyophilisiert.

Das Lyophilisat enthält nach Rekonstitution mit 1 ml H₂O bidest. folgende Bestandteile in der angegebenen Konzentration:

```
Apolipoprotein A1          127,0  mg/dl
Apolipoprotein A2           47,0  mg/dl
Apo B                      110,0  mg/dl.
```

## Ansprüche

1. Kontroll- bzw. Eichserum für die Lipid-Diagnostik, dadurch gekennzeichnet, daß es die für die Lipid-Diagnostik relevanten Parameter Apolipoproteine A1, A2 und B menschlichen Ursprungs im normalen oder pathologischen Konzentrationsbereich in einer einzigen, lagerstabilen Darreichungsform enthält.

2. Kontroll- bzw. Eichserum gemäß Anspruch 1, dadurch gekennzeichnet, daß es zusätzlich HDL-Cholesterin und LDL-Cholesterin enthält.

3. Kontroll- bzw. Eichserum gemäß Anspruch 1 oder 2, dadurch gekennzeichnet, daß es zusätzlich Gesamtcholesterin und Triglyceride enthält.

4. Kontroll- bzw. Eichserum gemäß einem der Ansprüche 1 bis 3, dadurch gekennzeichnet, daß es in lyophilisierter oder mit wässrigem Medium rekonstituierter Form vorliegt.

5. verfahren zur Herstellung eines Kontroll- bzw. Eichserums gemäß einem der Ansprüche 1 bis 4, dadurch gekennzeichnet, daß man entweder von frischem Humanserum oder von frischem Humanplasma, das zu Humanserum umgearbeitet wird, ausgeht, die gewünschte Parameter-Konzentration einstellt, gegebenenfalls mit Hilfsmitteln versetzt und in üblicher Weise in eine handelsfähige Form überführt.

6. verfahren gemäß Anspruch 5, dadurch gekennzeichnet, daß das frische Humanserum bzw. das frische Humanplasma unmittelbar nach dem Gewinnen tiefgefroren und erst unmittelbar vor dem Weiterverarbeiten aufgetaut wird.

7. Verfahren zur Herstellung eines Kontroll- bzw. Eichserums gemäß einem der Ansprüche 1 bis 4, dadurch gekennzeichnet, daß man von Rinderserumalbumin ausgeht, dieses mit geeigneten Parameter-Konzentraten bis zur gewünschten Konzentration vermischt, gegebenenfalls Hilfsmittel zusetzt und in üblicher Weise in eine handelsfähige Form überführt.

8. Verfahren gemäß Anspruch 7, dadurch gekennzeichnet, daß als Parameter-Konzentrate ein LDL-Cholesterin-Konzentrat, ein HDL-Cholesterin-Konzentrat und ein triglyceridreicher Eigelbextrakt verwendet werden.

9. Verfahren gemäß einem der Ansprüche 5 bis 8, dadurch gekennzeichnet, daß als Hilfsmittel 4 - 12 Gew.-% Saccharose zugesetzt werden.

## Claims

1. Control or calibration serum for lipid diagnosis, characterised in that it contains the parameters apolipoproteins A1, A2 and B of human origin relevant for lipid diagnosis in the normal or pathological concentration range in a single, storage-stable form of administration.

2. Control or calibration serum according to claim 1, characterised in that it additionally contains HDL cholesterol and LDL cholesterol.

3. Control or calibration serum according to claim 1 or 2, characterised in that it additionally contains total cholesterol and triglycerides.

4. Control or calibration serum according to one of claims 1 to 3, characterised in that it is present in lyophilised form or in a form reconstituted with an aqueous medium.

5. Process for the preparation of a control or calibration serum according to one of claims 1 to 4, characterised in that one starts from either fresh human serum or from fresh human plasma worked up to human serum, adjusts the desired parameter concentration, optionally mixes with adjuvants and converts in the usual way into a commercially useful form.

6. Process according to claim 5, characterised in that the fresh human serum or the fresh human plasma is, immediately after being obtained, deep frozen and first thawed immediately before the further working up.

7. Process for the preparation of a control or calibration serum according to one of claims 1 to 4, characterised in that one starts from bovine serum albumin, mixes this with suitable parameter concentrates up to the desired concentration, possibly adds adjuvants thereto, and converts in the usual way into a commercially useful form.

8. Process according to claim 7, characterised in that, as parameter concentrates, there are used an LDL cholesterol concentrate, an HDL cholesterol concentrate and a triglyceride-rich egg yolk extract.

9. Process according to one of claims 5 to 8, characterised in that, as adjuvant, there are added 4 - 12 wt.% of saccharose.


**Revendications**

1. Sérum témoin ou d'étalonnage pour le diagnostic des lipides, caractérisé en ce qu'il contient les paramètres caractéristiques pour le diagnostic des lipides, les apolipoprotéines A1, A2, et B d'origine humaine, en des gammes de concentrations normales ou pathologiques, sous une forme de présentation unique, stable au stockage.

2. Sérum témoin ou d'étalonnage selon la revendication 1, caractérisé en ce qu'il contient en outre le cholestérol HDL et le cholestérol LDL.

3. Sérum témoin ou d'étalonnage selon la revendication 1 ou 2, caractérisé en ce qu'il contient en outre le cholestérol total et les triglycérides.

4. Sérum témoin ou d'étalonnage selon les revendications 1 à 3, caractérisé en ce qu'il se présente sous forme lyophilisée ou sous forme reconstituée dans un milieu aqueux.

5. Procédé pour la préparation d'un sérum témoin ou d'étalonnage selon les revendications 1 a 4, caractérisé en ce que l'on part soit de sérum humain frais, soit de plasma humain frais qui a été transformé en sérum humain, on ajuste la concentration voulue en paramètre ajoute éventuellement des adjuvants et transforme, de manière usuelle, en une forme apte à la commercialisation.

6. Procédé selon là revendication 5, caractérisé en ce que le sérum humain frais ou le plasma humain frais sont congelés immédiatement après l'obtention et ne sont décongelés qu'immédiatement avant la transformation ultérieure.

7. Procédé pour la préparation d'un sérum témoin ou d'étalonnage selon les revendications 1 à 4, caractérisé en ce que l'on part d'albumine de sérum de boeuf, que l'on mélange celle-ci avec du concentré de paramètre approprié en l'ajustant à la concentration voulue, ajoute éventuellement des adjuvants et transforme le produit en une forme apte à la commercialisation, de manière usuelle.

8. Procédé selon la revendication 7, caractérisé en ce que comme concentré de paramètre, on peut utiliser un concentré de cholestérol LDL, un concentré de cholestérol HDL et un extrait de jaune d'oeuf riche en triglycérides.

9. Procédé selon l'une des revendications 5 à 9, caractérisé en ce que comme adjuvant, on ajoute 4-12 % en poids de saccharose.